(19) Europäisches Patentamt
European Patent Office
Office européen des brevets

(11) EP 4 134 107 B1

(12) EUROPÄISCHE PATENTSCHRIFT

(45) Veröffentlichungstag und Bekanntmachung des Hinweises auf die Patenterteilung:
**16.04.2025 Patentblatt 2025/16**

(21) Anmeldenummer: **21191103.7**

(22) Anmeldetag: **12.08.2021**

(51) Internationale Patentklassifikation (IPC):
***A61L 15/22*** *(2006.01)* ***A61L 15/26*** *(2006.01)*
***A61L 15/28*** *(2006.01)* ***A61L 15/64*** *(2006.01)*

(52) Gemeinsame Patentklassifikation (CPC):
(C-Sets verfügbar)
**A61L 15/225; A61L 15/26; A61L 15/28;**
**A61L 15/64;** A61L 2300/608 (Forts.)

(54) **WUNDAUFLAGE MIT EINER HYDROGELSCHICHT UND EINER WUNDKONTAKTSCHICHT UMFASSEND HYALURONSÄURE**

WOUND DRESSING COMPRISING A HYDROGEL LAYER AND A WOUND CONTACT LAYER COMPRISING HYALURONIC ACID

PANSEMENT DOTÉ D'UNE COUCHE D'HYDROGEL ET D'UNE COUCHE DE CONTACT AVEC LA PLAIE COMPRENANT DE L'ACIDE HYALURONIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**15.02.2023 Patentblatt 2023/07**

(73) Patentinhaber: **Paul Hartmann AG**
**89522 Heidenheim (DE)**

(72) Erfinder: **KETTEL, Dr., Markus**
**89520 Heidenheim (DE)**

(74) Vertreter: **Paul Hartmann AG**
**Patents & Licensing**
**Paul-Hartmann-Straße 12**
**89522 Heidenheim (DE)**

(56) Entgegenhaltungen:
**WO-A1-2010/000451 US-A1- 2002 111 576**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

(Forts. nächste Seite)

(52) Gemeinsame Patentklassifikation (CPC): (Forts.)

C-Sets
**A61L 15/225, C08L 5/08;**
**A61L 15/225, C08L 75/04**

## Beschreibung

Die vorliegende Erfindung betrifft eine Wundauflage umfassend eine Hydrogelschicht und eine Wundkontaktschicht. Die Wundkontaktschicht ist mit einer wundzugewandten Seite der Hydrogelschicht verbunden. Zudem betrifft die vorliegende Erfindung ein Herstellungsverfahren sowie eine Anwendung der zuvor genannten Wundauflage.

Die WO 2010/000451 A1 offenbart eine mehrschichtige Wundauflage umfassend eine Wundkontaktschicht und eine absorbierende Schicht. Die absorbierende Schicht umfasst einen hydrophilen Polyurethanschaum, welcher einen Wasseranteil von mindestens 10 Gew.-% aufweist. Die Wundkontaktschicht kann ein Hydrogel umfassen. Die in der WO'451 offenbarte Wundauflage verklebt nicht mit der Wunde und kann ein wundheilungsförderndes feuchtes Wundklima erzeugen, indem die Wundauflage sowohl überschüssige Feuchtigkeit von der Wunde aufnehmen als auch bei Bedarf Feuchtigkeit an die Wunde abgeben kann.

WO 2018/115257 A1 und EP 3587456 A1 beziehen sich auf Weiterentwicklungen der in WO'451 offenbarten Wundauflage. Gemäß WO'257 enthält die Hydrogel-Wundkontaktschicht nun als Feuchthaltemittel anstatt Propylenglykol insbesondere Glycerol. Dadurch besitzt das Hydrogel eine bessere Zellkompatibilität. Gemäß EP'456 enthält die Hydrogel-Wundkontaktschicht Chitosan. Damit wirkt das Hydrogel antibakteriell und kann vorteilhaft zur Behandlung von infizierten Wunden eingesetzt werden.

Die in WO'451, WO'257 und EP'456 offenbarten Wundauflagen ermöglichen in vielen Fällen eine schonende und effektive Wundbehandlung. Dennoch wäre es wünschenswert, wenn die Wundauflagen den Wundheilungsprozess noch wirksamer unterstützen könnten.

US 2002/0111576 A1 offenbart eine Wundauflage, umfassend:

(a) eine erste Schicht, die angrenzend an die Wunde angeordnet ist und die ein Material umfasst, das bioabsorbierbar, porös und dazu geeignet ist, als Gerüst für Zellanhaftung und -proliferation zu dienen; und
(b) eine zweite Schicht, die in Kontakt mit der ersten Schicht steht und die ein absorbierendes, gelbildendes Material umfasst, das dazu geeignet ist, als Barriere für Zelladhäsion und -penetration zu dienen.

Die Aufgabe der vorliegenden Erfindung bestand darin, eine verbesserte Wundauflage bereitzustellen. Insbesondere war es beabsichtigt, dass die Wundauflage das Potenzial hat den Wundheilungsprozess besonders wirksam unterstützen zu können. Gelöst werden diese Aufgaben mit einer Wundauflage nach Anspruch 1 und einem Verfahren nach Anspruch 19.

Erfindungsgemäß umfasst die Wundauflage eine Hydrogelschicht und eine Wundkontaktschicht. Die Wundkontaktschicht ist dabei mit einer wundzugewandten Seite der Hydrogelschicht verbunden. Die erfindungsgemäße Wundauflage ist dadurch gekennzeichnet, dass die Wundkontaktschicht Hyaluronsäure umfasst.

Die Hydrogelschicht der erfindungsgemäßen Wundauflage kann sowohl Feuchtigkeit aufnehmen als auch Feuchtigkeit abgeben. Somit kann die Wundauflage ein wundheilungsförderndes feuchtes Wundklima bei einer Vielzahl unterschiedlicher Wundtypen erzeugen.

Die Wundkontaktschicht der erfindungsgemäßen Wundauflage enthält einen Wirkstoff in Form der Hyaluronsäure, welcher vielfältige wundheilungsfördernde Eigenschaften auch auf biologischer beziehungsweise zellulärer Ebene haben kann. Die wundheilungsfördernden Eigenschaften der Hyaluronsäure sind in der wissenschaftlichen Literatur vielfach beschrieben und beispielsweise in dem Artikel "Hyaluronan in wound healing: Rediscovering a major player" (Wound Rep Reg (2014), 22, 579-593) zusammengefasst. So kann Hyaluronsäure unter anderem die Migration und Proliferation von an der Wundheilung beteiligten Zellen wie Fibroblasten stimulieren. Entsprechend kann die Wundauflage den Wundheilungsprozess gegenüber herkömmlichen Hydrogel-haltigen Wundauflagen weiter beschleunigen und unterstützen, so dass sich die Wunde schneller schließen und die Wunde schneller verheilen kann. Dabei kann auch eine unter kosmetischen Gesichtspunkten verbesserte Wundheilung stattfinden, zum Beispiel indem die Narbenbildung reduziert wird. Weitere Vorteile der Erfindung ergeben sich aus den nachfolgend beschriebenen bevorzugten Ausführungsformen der Erfindung.

Bevorzugt umfasst die Hydrogelschicht ein Polyurethan-Polymer, insbesondere ein Polyurethan-Polyharnstoff-Copolymer. Besonders bevorzugt besteht die Hydrogelschicht aus einem Polyurethan-Polymer, insbesondere einem Polyurethan-Polyharnstoff-Copolymer, als feste Phase und einer wässrigen Flüssigkeit als flüssige Phase. Derartige Hydrogele können besonders vorteilhaft in der Wundbehandlung eingesetzt werden.

Weiterhin ist die Hydrogelschicht vorzugsweise erhältlich durch Umsetzung eines Amin-terminierten Präpolymers enthaltend Polyalkylenoxideinheiten mit einem Isocyanat-terminierten Präpolymer enthaltend Polyalkylenoxideinheiten in Gegenwart von Wasser und optional eines mehrwertigen Alkohols, insbesondere Glycerol. Bei einer solchen Umsetzung können die zuvor genannten vorteilhaften Hydrogele mit einem Polyurethan-Polymer beziehungsweise Polyurethan-Polyharnstoff-Copolymer gebildet werden. Die Umsetzung kann auch in Gegenwart von weiteren Komponenten stattfinden. Insbesondere kann die Umsetzung auch in Gegenwart eines oder mehrerer Salze ausgewählt aus der Gruppe bestehend aus Natriumchlorid, Kaliumchlorid, Magnesiumchlorid und Calciumchlorid stattfinden. Aus dieser

Gruppe anorganischer Salze wird Natriumchlorid besonders bevorzugt.

Vorzugsweise beträgt bei dieser Umsetzung die Summe der Massen aus Amin-terminierten Präpolymer und Isocyanat-terminierten Präpolymer 10 bis 30 Gew.-%, die Masse des mehrwertigen Alkohols - falls vorhanden - 5 bis 35 Gew.-% und die Masse des Wassers mindestens 40 Gew.-%. Die Gewichtsangaben beziehen sich dabei auf die Gesamtmasse aller Reaktanden. Zudem kann bei der genannten Umsetzung das molare Verhältnis reaktiver Isocyanatendgruppen zu reaktiven Aminendgruppen 1,0 bis 1,5 betragen. Weitergehende Angaben zur Herstellung derartiger Hydrogele sind in der WO 2018/115257 A1 enthalten.

Die Polyalkylenoxideinheiten der beiden zuvor erwähnten Präpolymere können durch Polyethylenoxid- und/oder Polypropylenoxideinheiten gebildet werden, wobei das Gewichtsverhältnis von Ethylenoxid- zu Propylenoxideinheiten vorzugsweise 3 : 1 bis 7 : 1 beträgt. Darüber hinaus ist das Isocyanat-terminierte Präpolymer vorteilhafterweise mindestens dreiarmig verzweigt. Insbesondere ist das Isocyanat-terminierte Präpolymer genau dreiarmig verzweigt.

Insbesondere handelt es sich bei dem Amin-terminierten Präpolymer um ein Triblock-Polymer aus Propylenoxid-, Ethylenoxid- und wieder Propylenoxideinheiten, wobei das Polymer endständig jeweils mit 2-Aminopropylgruppen aminfunktionalisiert ist. Es weist typischerweise einen Gehalt reaktiver Aminendgruppen von 0,9554 mmol/g bei einem Molekulargewicht von durchschnittlich etwa 2000 g/mol und einer Dispersität von 1,08, gemessen durch Gelpermeationschromatographie, auf. Das Gewichtsverhältnis von Ethylenoxid- zu Propylenoxideinheiten beträgt 3 : 1 bis 7 : 1, bevorzugt 39 : 6. Ein solches Amin-terminiertes Präpolymer ist beispielsweise als Jeffamin® ED-2003 (Huntsman; Everberg, Belgien) im Handel erhältlich.

Insbesondere handelt es sich bei dem Isocyanat-terminierten Präpolymer um ein dreiarmiges Copolymer aus Ethylenoxid- und Propylenoxideinheiten, welches endständig jeweils mit einem Molekül Isophorondiisocyanat umgesetzt wurde. Es weist üblicherweise einen Gehalt reaktiver Isocyanatendgruppen (NCO-Gruppen) von 2,5 % bis 4,0 %, bevorzugt 3,0 % bis 3,4 %, besonders bevorzugt 3,2 %, und ein Gewichtsverhältnis von Ethylenoxideinheiten zu Propylenoxideinheiten von 3 : 1 bis 4 : 1 auf. Ein derartiges Isocyanat-terminiertes Präpolymer mit aliphatischen Isocyanat-Gruppen ist zum Beispiel als Aquapol® PI-13000-31 (Carpenter; Richmond, USA) kommerziell erhältlich.

Die nachfolgende Abbildung veranschaulicht die schematische Struktur eines dreiarmig verzweigten Isocyanat-terminierten Präpolymers enthaltend Polyethylenoxid- und Polypropylenoxideinheiten (wie bei Aquapol®). Ein Glycerol-Molekül bildet das Zentrum des Präpolymers. Die drei "Arme" des Präpolymers mit jeweils einer endständigen Isocyanat-Gruppe sind mit den Hydroxyl-Gruppen des Glycerol-Moleküles verknüpft. Das Glycerol-Molekül selbst ist in der Abbildung nicht dargestellt. Es würde sich in der rechten Bildhälfte dort befinden, wo die als Wellenlinie schematisiert dargestellten drei "Arme" aufeinandertreffen. In der linken Bildhälfte ist die chemische Struktur eines "Arms" genauer dargestellt.

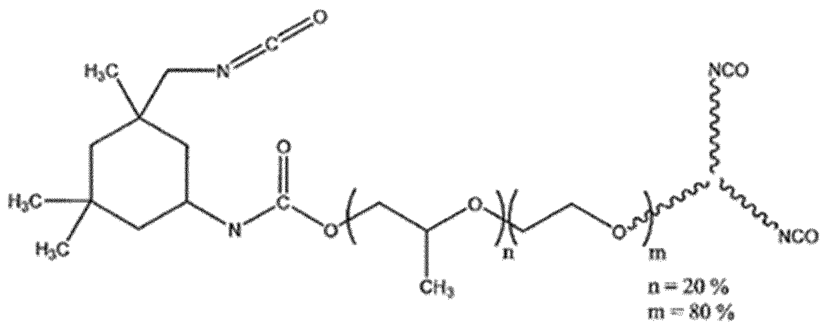

n = 20 %
m = 80 %

In einer bevorzugten Ausführungsform der Erfindung ist die Hydrogelschicht perforiert. Die Perforationen können die Hydrogelschicht durchlässiger für Wundexsudat machen, was insbesondere dann vorteilhaft sein kann, wenn die Wundauflage eine zusätzliche absorbierende Schicht umfasst. Die Schichtdicke der Hydrogelschicht kann beispielsweise 0,5 bis 5 mm, bevorzugt 0,5 bis 3 mm, betragen.

Kennzeichnend und wesentlich für die vorliegende Erfindung ist die Hyaluronsäure, welche in der Wundkontaktschicht enthalten ist. Sie liegt in der Wundkontaktschicht typischerweise im Wesentlichen frei, also in nicht quervernetzter Form, vor. Für die Erfindung kann auch ein Salz der Hyaluronsäure verwendet werden. Somit kann die erfindungsgemäße Wundkontaktschicht genauso gut ein Salz der Hyaluronsäure umfassen. Bei dem Salz kann es sich beispielsweise um das Kaliumsalz oder auch das Natriumsalz der Hyaluronsäure handeln. Im Handel sind Hyaluronsäuren beziehungsweise Salze der Hyaluronsäure aus verschiedenen Quellen und in verschiedenen molekularen Gewichten verfügbar. Die Hyaluronsäure kann ein molekulares Gewicht von weniger als 1000 kDa (niedriges molekulares Gewicht), bevorzugt 1000 bis 1800 kDa (mittleres molekulares Gewicht) oder besonders bevorzugt mehr als 1800 kDa (hohes molekulares Gewicht) aufweisen. Hyaluronsäure mit einem niedrigen molekularen Gewicht ist gegebenenfalls besser wasserlöslich und lässt sich deshalb möglicherweise besser verarbeiten. Dennoch ist Hyaluronsäure mit einem mittleren oder hohen molekularen Gewicht bevorzugt, da sie die Wundheilung besonders positiv beeinflussen kann.

Insbesondere ist es im Rahmen der vorliegenden Erfindung vorgesehen, dass die Wundkontaktschicht als Opferschicht ausgebildet ist, so dass sich die Wundkontaktschicht bei Kontakt mit Wundexsudat zumindest teilweise auflösen und/oder die Wundkontaktschicht vom Wundgewebe zumindest teilweise resorbiert werden kann. Insbesondere kann die als Opferschicht ausgebildete Wundkontaktschicht im Wesentlichen vollständig bei Kontakt mit Wundexsudat aufgelöst und/oder im Wesentlichen vollständig vom Wundgewebe resorbiert werden. Nachdem die Wundkontaktschicht aufgelöst und/oder resorbiert ist, kann die Hydrogelschicht die Wundkontaktschicht der Wundauflage bilden. Das Hydrogel als neue Wundkontaktschicht verklebt nicht mit der Wunde und ermöglicht dadurch einen schonenden Verbandswechsel. Damit ist also vorgesehen, dass die Hyaluronsäure von der Wundauflage an die Wunde abgegeben und die Hydrogelschicht im Laufe der Wundbehandlung die Wundkontaktschicht bilden kann, wobei besonders gute wundheilungsfördernde Effekte erzielt werden können.

Die Wundkontaktschicht kann eine Hyaluronsäurefreisetzung von mindestens 50 %, bevorzugt von mindestens 60 %, mehr bevorzugt von mindestens 70 %, besonders bevorzugt von mindestens 80 % und ganz besonders bevorzugt von mindestens 90 % aufweisen. Die Hyaluronsäurefreisetzung kann dabei mit der im vorliegenden Dokument beschriebenen Testmethode bestimmt werden und sich auf eine Inkubationszeit von drei Tagen beziehen. Der Ablauf der Testmethode ist im zweiten Punkt der Versuchsergebnisse beschrieben.

Erfindungsgemäß ist die Wundkontaktschicht filmartig beziehungsweise folienartig ausgebildet. Eine solche filmartige Wundkontaktschicht kann die Hyaluronsäure in vorteilhafter Weise freisetzen und ist einfach herzustellen. Zudem kann eine solche filmartige Wundkontaktschicht leicht mit der Hydrogelschicht verbunden werden. Weiterhin ist das Material, aus dem die Wundkontaktschicht besteht, nicht porös ausgebildet, damit sich die Wundkontaktschicht bei Kontakt mit Wundexsudat nicht zu schnell auflöst. Eine nicht poröse Wundkontaktschicht kann die Hyaluronsäure also gegebenenfalls gleichmäßiger und über einen längeren Zeitraum freisetzen. Die Schichtdicke der Wundkontaktschicht kann 0,1 bis 3,0 mm, bevorzugt 0,1 bis 1,5 mm und besonders bevorzugt 0,1 bis 1,0 mm betragen. Dabei kann die angegebene untere Grenze für die Schichtdicke von 0,1 mm auch höher angesetzt werden und zum Beispiel 0,2 mm, 0,3 mm, 0,4 mm oder 0,5 mm betragen. Ein weiterer Vorteil der filmartigen sowie nicht porösen Wundkontaktschicht ist, dass sie ebenso wie die Hydrogelschicht transparent ausgebildet sein kann.

Gemäß einer ganz besonders bevorzugten Ausführungsform der Erfindung ist die Wundkontaktschicht erhältlich durch Trocknen einer flüssigen oder gelartigen Zubereitung umfassend Hyaluronsäure und Wasser. Damit kann eine als Opferschicht ausgebildete, filmartige und nicht poröse transparente Wundkontaktschicht erhalten werden.

Das Trocknen kann zum Beispiel eine Gefriertrocknung und/oder eine Lufttrocknung umfassen. Allerdings kann die Gefriertrocknung zu einer porösen Wundkontaktschicht führen und wird deshalb vorliegend weniger bevorzugt. Entsprechend stellt die Lufttrocknung vorliegend die bevorzugte Trocknungsmethode dar. Die Lufttrocknung kann auch mit einem technischen Hilfsmittel, beispielsweise einem Trockenschrank, durchgeführt werden, um den Trocknungsprozess zu beschleunigen.

Ferner ist es bevorzugt, wenn die Zubereitung nur teilweise getrocknet ist, so dass ein Rest Wasser in der Wundkontaktschicht vorhanden ist. Die Wundkontaktschicht kann dann flexibler und reißfester sein. Zum Beispiel kann die Wundkontaktschicht bis zu 50 Gew.-%, bis zu 40 Gew.-%, bis zu 30 Gew.-%, bis zu 20 Gew.-%, bis zu 10 Gew.-% oder nur bis zu 5 Gew.-% Wasser, bezogen auf das Gesamtgewicht der Wundkontaktschicht, enthalten.

Wie zuvor erwähnt umfasst die für das Trocknen verwendete Zubereitung Hyaluronsäure und Wasser. Die Hyaluronsäurekonzentration der Zubereitung kann zum Beispiel 1 bis 10 Gew.-%, 1 bis 8 Gew.-%, 1 bis 6 Gew.-%, 1 bis 4 Gew.-% oder 1 bis 2 Gew.-%, bezogen auf das Gesamtgewicht der Zubereitung, betragen. Die Zubereitung kann auch noch eine oder mehrere weitere Komponenten wie zum Beispiel einen antiinflammatorischen oder antimikrobiellen Wirkstoff enthalten. Ebenso ist es aber auch möglich, dass keine derartigen weiteren Komponenten vorhanden sind und die Zubereitung entsprechend aus Hyaluronsäure und Wasser besteht. Eine solche Zubereitung ist einfach und kostengünstig herstellbar. Jedenfalls ist es im Sinne der vorliegenden Erfindung typischerweise vorgesehen, dass die Zubereitung keine Quervernetzer für die Hyaluronsäure enthält.

Vorteilhafterweise ist die Wundkontaktschicht perforiert. Diese Perforationen sollen ähnlich wie die Perforationen in der Hydrogelschicht die Durchlässigkeit für Wundexsudat erhöhen. Sie können auch den vorteilhaften Effekt haben, dass die Hydrogelschicht eine trockene Wunde besser mit Feuchtigkeit versorgen kann. Falls sowohl die Hydrogelschicht als auch die Wundkontaktschicht perforiert sind, können sich die Perforationen in der Hydrogelschicht und der Wundkontaktschicht vorzugsweise überlagern, so dass der Verbund aus Hydrogelschicht und Wundkontaktschicht durchgehende Perforationen aufweist, welche den ungehinderten Durchtritt von Wundexsudat durch den Verbund aus Hydrogelschicht und Wundkontaktschicht beispielsweise in eine zusätzlich vorhandene absorbierende Schicht der Wundauflage ermöglichen.

Die Perforationen in der Hydrogelschicht sowie in der Wundkontaktschicht können im Wesentlichen rund ausgebildet und in regelmäßigen Abständen in den Schichten vorhanden sein. Sie können zudem einen Durchmesser von 0,5 bis 5 mm, bevorzugt 1 bis 3 mm, aufweisen. Die Anzahl und die Größe der Perforationen können so gewählt sein, dass die Schichten netzartig ausbildet sind. Die Perforationen in der Hydrogelschicht sowie in der Wundkontaktschicht können durch Schneiden oder Stanzen erzeugt werden. Alternativ ist es möglich die Perforationen bei der Polymerisation

beziehungsweise dem Trocknen der Schichten durch geeignete Gießformen zu erzeugen. Zum Beispiel kann wie in WO 2010/000451 A1 beschrieben eine Form mit einer Noppenstruktur verwendet werden, um die Perforationen zu erzeugen.

Erfindungsgemäß ist die Wundkontaktschicht mit der wundzugewandten Seite der Hydrogelschicht verbunden. Die Wundkontaktschicht kann mit der Hydrogelschicht verbunden sein, indem die Schichten miteinander in Kontakt gebracht werden, wenn die Hydrogelschicht noch nicht vollständig polymerisiert ist. Alternativ kann die Wundkontaktschicht mit der Hydrogelschicht durch ein Verbindungsmittel verbunden sein. Überraschend hat sich gezeigt, dass als Verbindungsmittel auch ein Isocyanat-terminiertes Präpolymer enthaltend Polyalkylenoxideinheiten verwendet werden kann. Dieses Präpolymer wird wie vorangehend beschrieben bevorzugt zur Herstellung der Hydrogelschicht verwendet. Zur Herstellung der Hydrogelschicht und zur Verbindung der Wundkontaktschicht mit der Hydrogelschicht kann vorteilhafterweise das gleiche Isocyanat-terminierte Präpolymer verwendet werden. Das Verbindungsmittel ist dann aus chemischer Sicht besonders gut mit der Hydrogelschicht kompatibel.

Wenn die Wundkontaktschicht wie zuvor beschrieben mit der Hydrogelschicht verbunden wird, kann eine kovalente Verbindung der beiden Schichten erfolgen, indem ein Teil des in dem Hydrogel beziehungsweise dem Verbindungsmittel enthaltenen Isocyanat-terminierten Präpolymers mit einem Teil der in der Wundkontaktschicht enthaltenen Hyaluronsäure reagiert. Der kovalent an die Hydrogelschicht angebundene Teil der Wundkontaktschicht kann dann gegebenenfalls nicht mehr an die Wunde abgegeben werden und ein Bestandteil der Hydrogelschicht sein. Um möglichst viel Hyaluronsäure freisetzen zu können, wird deshalb vorgeschlagen, die Wundkontaktschicht und die Hydrogelschicht nur bereichsweise miteinander zu verbinden. Denkbar ist beispielsweise, das Verbindungsmittel nur punktuell zwischen der Wundkontaktschicht und der Hydrogelschicht vorzusehen.

Normalerweise umfasst die erfindungsgemäße Wundauflage außer der Hydrogelschicht und der Wundkontaktschicht noch weitere Schichten beziehungsweise Verbandslagen. Zum Beispiel umfasst die Wundlauflage üblicherweise weiterhin eine Rückschicht als äußerste, wundabgewandte Verbandslage. Die Rückschicht kann eine wasserundurchlässige und wasserdampfdurchlässige Kunststofffolie, insbesondere Polyurethanfolie, umfassen. Zudem kann eine wundzugewandte Seite der Rückschicht adhäsiv, beispielsweise mit einem Acrylatkleber, beschichtet sein. Vorteilhafterweise überfängt die Rückschicht in diesem Fall die weiteren Schichten der Wundauflage und bildet einen Kleberand aus, wobei die Wundauflage dann in der Art eines Inselverbandes ausgestaltet sein kann. Mit dem Kleberand kann die Wundauflage am Körper des Patienten befestigt werden. Die Rückschicht kann mit einer wundabgewandten Seite der Hydrogelschicht verbunden sein.

Außerdem ist besonders bevorzugt vorgesehen, dass die Wundauflage weiterhin eine absorbierende Schicht, insbesondere eine absorbierende Schaumstoffschicht, umfasst.

Damit kann die Wundauflage mehr Flüssigkeit aufnehmen. Die Wundauflage ist dann insbesondere auch zur Behandlung von stärker exsudierenden Wunden geeignet. Besonders geeignet ist eine Schaumstoffschicht in der Form eines hydrophilen Polyurethanschaums, welche vorteilhafterweise einen Wasseranteil von mindestens 10 Gew.-% umfassen kann. Die absorbierende Schicht ist bevorzugt mit einer wundabgewandten Seite der Hydrogelschicht verbunden. Die üblicherweise vorhandene Rückschicht ist dann mit einer wundabgewandten Seite der absorbierenden Schicht verbunden, so dass die absorbierende Schicht zwischen der Rückschicht und der Hydrogelschicht angeordnet ist. Die Schichtdicke der absorbierenden Schicht kann zum Beispiel 0,5 bis 5 mm, bevorzugt 0,5 bis 3 mm, betragen. Weitere mögliche Merkmale und Vorteile der absorbierenden Schicht sind in der WO 2010/000451 A1 offenbart.

Die Erfindung betrifft auch ein Verfahren zur Herstellung der erfindungsgemäßen Wundauflage umfassend die nachfolgenden Schritte:

i. Bereitstellen einer Hydrogelschicht,
ii. Bereitstellen einer Wundkontaktschicht umfassend Hyaluronsäure,
iii. Verbinden der Wundkontaktschicht mit einer wundzugewandten Seite der Hydrogelschicht.

Das Bereitstellen der Wundkontaktschicht kann die folgenden Schritte umfassen:

i. Lösen von Hyaluronsäure in einer wässrigen Lösung, wobei eine Hyaluronsäure-haltige Zubereitung gebildet wird,
ii. Trocknen der Hyaluronsäure-haltigen Zubereitung, wobei eine Hyaluronsäure-haltige Schicht gebildet wird.

Für Schritt ii. kann die Hyaluronsäure-haltige Zubereitung zum Beispiel in eine Petrischale überführt oder auf einen Folienträger aufgetragen und dann einer Lufttrocknung ausgesetzt werden.

Die erfindungsgemäße Wundauflage ist besonders gut zur Behandlung von Wunden am menschlichen oder tierischen Körper in der Granulations- oder Epithelisierungsphase geeignet. Entsprechend richtet sich die Erfindung auch auf eine Verwendung der erfindungsgemäßen Wundauflage zur Behandlung von Wunden am menschlichen oder tierischen Körper in der Granulations- oder Epithelisierungsphase. Mit anderen Worten richtet sich die Erfindung also auch auf die erfindungsgemäße Wundauflage zur Anwendung in der Behandlung von Wunden am menschlichen oder tierischen Körper in der Granulations- oder Epithelisierungsphase. Der Erfindungsgegenstand umfasst gleichfalls die

therapeutische Verwendung der in der Wundauflage enthaltenen Hyaluronsäure zur Wundheilung, insbesondere zur phasengerechten Wundheilung. Demnach richtet sich die Erfindung dann auf Hyaluronsäure zur Anwendung in der Behandlung von Wunden am menschlichen oder tierischen Körper in der Granulations- oder Epithelisierungsphase, wobei die Hyaluronsäure in der erfindungsgemäßen Wundauflage enthalten ist.

Das Herstellungsverfahren und die Verwendung der erfindungsgemäßen Wundauflage beziehen sich auch auf die voranstehend beschriebenen speziellen Ausführungsformen der Wundauflage. Das heißt, die weiteren Merkmale der voranstehend beschriebenen speziellen Ausführungsformen der Wundauflage sind auch Bestandteil des Herstellungsverfahrens und der Verwendung der Wundauflage.

## Beispiele und Figuren

Mit den nachfolgend beschriebenen Beispielen und Figuren soll die Erfindung exemplarisch näher erläutert und veranschaulicht werden. Dabei können in den Figuren ähnliche Strukturelemente mit denselben Bezugszeichen ausgewiesen sein.

**Figur 1** zeigt eine Schnittansicht einer erfindungsgemäßen Wundauflage **1** in einer ersten Ausführungsform. Die Wundauflage **1** umfasst eine Hydrogelschicht **2,** welche bevorzugt aus einem Polyurethan-Polyharnstoff-Hydrogel besteht. Die Wundauflage **1** umfasst weiterhin eine Wundkontaktschicht **3,** welche mit einer wundzugewandten Seite der Hydrogelschicht **2** verbunden ist. Die Wundkontaktschicht **3** umfasst Hyaluronsäure und ist als "Opferschicht" ausgebildet. "Opferschicht" bedeutet vorliegend, dass die Wundkontaktschicht **3** im Laufe der Wundbehandlung zumindest teilweise oder vorzugsweise im Wesentlichen vollständig aufgelöst und/oder resorbiert werden kann, wobei die Hyaluronsäure von der Wundkontaktschicht **3** als wundheilungsfördernder Wirkstoff an die Wunde abgegeben werden kann. Wenn sich die Wundkontaktschicht **3** aufgelöst hat, kann die Hydrogelschicht **2** in unmittelbaren Kontakt mit der Wundoberfläche treten. Sie stellt dann also die neue Wundkontaktschicht der Wundauflage **1** dar. Schließlich umfasst die Wundauflage **1** noch eine mit einer wundabgewandten Seite der Hydrogelschicht **2** verbundene Rückschicht **4.** Diese grenzt die Wundauflage **1** nach außen hin ab und schützt die Wundauflage **1** insbesondere vor Kontamination. Die Rückschicht **4** besteht aus einer wasserundurchlässigen und wasserdampfdurchlässigen Kunststofffolie, zum Beispiel einer Polyurethanfolie. Die Wundauflage **1** kann in vorteilhafter Weise transparent ausgebildet sein, um den Zustand der Wunde während der Behandlung beobachten zu können. In der Anwendung wird die Wundauflage **1** auf die Wunde gelegt und gegebenenfalls mit einem zusätzlichen Verbandsmaterial wie beispielsweise einer Binde oder einem Filmverband fixiert.

Die Wundauflage **1** kann hergestellt werden, indem die Rückschicht **4** mit einer Reaktionsmischung beschichtet wird, die nach Polymerisation die Hydrogelschicht **2** ausbildet. Die insbesondere durch eine Lufttrocknung einer wässrigen Hyaluronsäure-haltigen Zubereitung erhältliche Wundkontaktschicht **3** kann dann mit einem Verbindungsmittel, bevorzugt einem Isocyanat-terminierten Präpolymer, wie vorangehend bereits beschrieben mit der Hydrogelschicht **2** verbunden werden.

**Figur 2** zeigt eine Schnittansicht einer erfindungsgemäßen Wundauflage **5** in einer zweiten Ausführungsform. Die Wundauflage **5** unterscheidet sich von der Wundauflage **1** aus **Figur 1** dadurch, dass die Wundkontaktschicht **3** perforiert ist. Die Perforationen **6** können sowohl die Aufnahme von Exsudat von der Wunde als auch die Abgabe von Feuchtigkeit an die Wunde durch die Hydrogelschicht **2** verbessern, indem die Durchlässigkeit der Wundkontaktschicht **3** für Flüssigkeit erhöht ist. Diese Verbesserung kommt insbesondere am Beginn der Wundbehandlung zum Tragen, wenn sich die Wundkontaktschicht **3** noch nicht aufgelöst hat. Die Perforationen **6** können somit den Erhalt eines wundheilungsfördernden feuchten Wundklimas beschleunigen und fördern.

In **Figur 3** ist die Wundauflage **5** mit Blick auf die Unterseite dargestellt. Wie aus **Figur 3** hervorgeht, sind die Perforationen **6** in einem regelmäßigen Muster in der Wundkontaktschicht **3** vorhanden und rund ausgebildet. Die Perforationen **6** können bevorzugt einen Durchmesser von 1 bis 3 mm aufweisen. Die Perforationen **6** können jedoch auch größer und/oder zahlreicher als in **Figur 3** gezeigt ausgebildet sein, so dass die Wundkontaktschicht **3** eine netzartige Struktur aufweisen kann.

**Figur 4** zeigt eine Schnittansicht einer erfindungsgemäßen Wundauflage **7** in einer dritten Ausführungsform. Zusätzlich zu den drei Schichten **2, 3** und **4** der Wundauflage **1** weist die Wundauflage **7** eine vollflächig adhäsiv ausgerüstete weitere Verbandslage **8, 9** auf. Das Bezugszeichen **8** richtet sich dabei auf das Adhäsiv, bei dem es sich vorzugsweise um ein Acrylatkleber handeln kann. Bezugszeichen **9** richtet sich dann auf die Trägerschicht der zusätzlichen Verbandslage. Bei der Trägerschicht **9** handelt es sich ebenso wie bei der Schicht **4** um ein wasserundurchlässiges, jedoch wasserdampfdurchlässiges Folienmaterial aus beispielsweise Polyurethan. Die neue Verbandslage **8, 9** ist mittels dem Adhäsiv **8** an der wundabgewandten Seite der Schicht **4** befestigt, so dass die Verbandslage **8, 9** beziehungsweise deren Schicht **9** nun als die eigentliche Rückschicht der Wundauflage **7** fungiert. Wie in **Figur 4** sowie insbesondere **Figur 5** (Blick auf die Unterseite der Wundauflage **7**) dargestellt, überfängt die Verbandslage **8, 9** die Schichten **2, 3** und **4** der Wundauflage **7** und bildet einen Kleberand **10** aus. Mit dem Kleberand **10** kann die Wundauflage **7** dauerhaft und einfach am Körper des Patienten befestigt werden, ohne dass dafür ein separater Sekundärverband benötigt wird. Die Wundauflage **7** kann wie die Wundauflage **5** eine perforierte Wundkontaktschicht **3** aufweisen und zudem transparent ausgebildet sein, so dass eine Inspektion der Wunde für den Patienten und das Pflegepersonal auch dann möglich ist, wenn die Wundauflage **7** die Wunde bedeckt.

**Figur 6** zeigt eine Schnittansicht einer erfindungsgemäßen Wundauflage **11** in einer vierten Ausführungsform. Die Wundauflage **11** enthält die Schichten **2, 3, 8** und **9** der Wundauflage **7.** Zwischen der adhäsiven Schicht **8** und der Hydrogelschicht **2** befindet sich bei dieser Ausgestaltung jedoch eine absorbierende Schicht **12,** bei der es sich insbesondere um eine absorbierende Schaumstoffschicht beispielsweise in Form eines hydrophilen Polyurethanschaumstoffs handeln kann. Durch die zusätzliche absorbierende Schicht **12** kann die Wundauflage **11** mehr Wundexsudat aufnehmen als die Ausführungsformen aus **Figur 1** bis **5.** Sie ist damit besser zu Behandlung von stärker exsudierenden Wunden geeignet. Die absorbierende Schicht **12** kann aber auch vorteilhaft bei der Behandlung von trockenen Wunden sein, nämlich dann wenn sie herstellerseitig mit einem Anteil Wasser oder einer anderen geeigneten Flüssigkeit wie isotonische Kochsalzlösung oder Ringerlösung beaufschlagt wird.

Die Wundauflage **11** kann hergestellt werden, indem die Oberfläche der wundzugewandten Seite der absorbierenden Schicht **12** mit der noch nicht vollständig polymerisierten Hydrogelschicht **2** in Kontakt gebracht wird, wobei die Hydrogelschicht **2** teilweise in die poröse absorbierende Schicht **12** eindringt und somit eine Verbindung der beiden Schichten erfolgt. Danach kann auf der wundabgewandten Seite der absorbierenden Schicht **12** die Klebefolie **8, 9** befestigt werden. Nähere Angaben zu diesen Schritten sind in der bereits mehrfach genannten WO 2010/000451 A1 enthalten. Schließlich kann die Wundkontaktschicht **3** wie zuvor im Zusammenhang mit Wundauflage **1** beschrieben mit der Hydrogelschicht **2** verbunden werden.

Die Wundauflage **11** kann vorteilhafterweise gleichfalls Perforationen **6** in der Wundkontaktschicht **3** aufweisen, wobei die Perforationen **6** dabei vorzugsweise auch die Hydrogelschicht **2** durchdringen. Dadurch kann Flüssigkeit jederzeit und ungehindert zwischen der Wunde und den Schichten **2, 3** sowie **12** ausgetauscht werden. Insbesondere kann überschüssiges Wundexsudat die Schichten **2** und **3** ungehindert passieren und von der absorbierenden Schicht **12** aufgenommen und gespeichert werden. Eine solche Ausgestaltung der Wundauflage ist in **Figur 7** im Schnitt sowie in **Figur 8** mit Blick auf die Unterseite gezeigt und mit dem Bezugszeichen **13** ausgewiesen.

Zum Schutz während der Lagerung und zur leichteren Applikation der Wundauflagen können diese zumindest noch eine Abdeckschicht aufweisen (nicht dargestellt). Die Abdeckschicht ist mit der Wundkontaktschicht lösbar verbunden und wird abgezogen, bevor die Wundauflage auf die Wunde gelegt wird. Applikationssysteme für Wundauflagen mit einer solchen Abdeckschicht sind im Stand der Technik umfassend beschrieben. Die Abdeckschicht kann zum Beispiel eine zweiteilige Folienschicht aus silikonisiertem Polypropylen umfassen.

Versuchsergebnisse

Es wurden mehrere Proben eines Verbundes aus einer Hydrogelschicht und einer Hyaluronsäure-haltigen Schicht hergestellt und im Hinblick auf die Hyaluronsäurefreisetzung, die Absorptionskapazität sowie den pH-Wert untersucht.

1. Herstellung der Proben

i. Es wurde eine Hydrogelschicht hergestellt, indem ein Amin-terminiertes Präpolymer enthaltend Polyalkylenoxideinheiten mit einem Isocyanat-terminierten Präpolymer enthaltend Polyalkylenoxideinheiten in Gegenwart von demineralisiertem Wasser, Glycerol und Natriumchlorid umgesetzt wurde. Als Amin-terminiertes Präpolymer kam dabei Jeffamin® ED-2003 (Huntsman; Everberg, Belgien) und als Isocyanat-terminiertes Präpolymer Aquapol® PI-13000-31 (Carpenter; Richmond, USA) zum Einsatz. Die Hydrogelschicht enthielt folglich ein Polyurethan-Polyharnstoff-Copolymer als feste Phase und eine Mischung aus Wasser, Glycerol sowie Natriumchlorid als flüssige Phase. Das hergestellte Hydrogel wies bezogen auf das Gesamtgewicht der eingesetzten Stoffe die folgende Zusammensetzung auf:

7,58 Gew.-% Amin-terminiertes Präpolymer (Jeffamin)
13,03 Gew.-% Isocyanat-terminiertes Präpolymer (Aquapol)
61,54 Gew.-% Wasser
16,85 Gew.-% Glycerol
0,99 Gew.-% Natriumchlorid

ii. Es wurde eine Hyaluronsäure-haltige Schicht hergestellt, indem Hyaluronsäure in demineralisiertem Wasser bis zur Sättigungsgrenze gelöst und die erhaltene Zubereitung in eine Petrischale überführt wurde. Die in der Petrischale

enthaltene Zubereitung wurde im Anschluss im Trockenschrank bei einer Temperatur von 55 °C für 3 Tage luftgetrocknet. Es bildete sich am Boden der Petrischale eine filmartige Schicht aus. Die verwendete Hyaluronsäure besaß ein molekulares Gewicht von 1800 kDa und stammte von der Firma Pharmasports GmbH & Co. KG (Bergen, Deutschland).

iii. Es wurden Probenstücke in einer Größe von circa 1 cm x 1 cm (Länge x Breite) aus der Hydrogelschicht sowie der Hyaluronsäure-haltigen Schicht gestanzt beziehungsweise geschnitten. Das Gewicht der einzelnen Probenstücke wurde bestimmt.

iv. Die Hydrogel-Probenstücke wurden auf einer Seite mit Hilfe eines Spatels mit dem verwendeten Isocyanat-terminierten Präpolymer (Aquapol® PI-13000-31) dünn und gleichmäßig bestrichen und mit den Hyaluronsäure-Probenstücken in Kontakt gebracht, wobei die gewünschten Proben mit der Hydrogelschicht und der Hyaluronsäure-haltigen Schicht erhalten wurden. Dabei führte das Präpolymer in der Art eines Klebstoffs die Verbindung der beiden Schichten herbei. Das Gewicht der einzelnen Proben wurde bestimmt. Von jeder einzelnen Probe war somit bekannt, welches Gewicht die von der Probe umfassten Schichten aufweisen sowie welches Gewicht die Probe insgesamt aufweist.

**Figur 9** und **10** zeigen beispielhaft eine der hergestellten Proben mit Blick von oben beziehungsweise von der Seite. Die untere Schicht ist das Hydrogel und die obere Schicht der Hyaluronsäure-Film, welcher in der Anwendung als Wundkontaktschicht vorgesehen ist. Wie aus den Figuren hervorgeht, ist die Probe transparent ausgebildet.

2. Hyaluronsäurefreisetzung der Proben

i. Eine wie zuvor beschrieben hergestellte Probe wurde in eine mit demineralisiertem Wasser gefüllte Petrischale mit bekanntem Gewicht gelegt und für 1 bis 7 Tage bei Raumtemperatur inkubiert. Dabei wurde darauf geachtet, dass sich zumindest die Hyaluronsäure-haltige Schicht der Probe stets unterhalb der Wasseroberfläche befand.

ii. Nach Ablauf der Inkubationszeit wurde die Probe aus der Petrischale entfernt. Die Petrischale mit der Flüssigkeit wurden dann solange im Trockenschrank bei 55 °C aufbewahrt, bis die Flüssigkeit vollständig verdampft war. Es verblieb ein Rückstand in der Petrischale, von dem angenommen wurde, dass er aus freigesetzter Hyaluronsäure sowie gegebenenfalls Glycerol und Natriumchlorid besteht.

iii. Die Petrischale wurde gewogen, um das Gewicht des eingetrockneten Rückstandes zu ermitteln. Die von der Probe freigesetzte Menge an Hyaluronsäure in Prozent (HAFp) wurde nach folgender Formel berechnet:

$$HAF_P = (m_{RP} - m_{RK}) / m_{HAS} * 100$$

Die Abkürzung $m_{RP}$ steht dabei für das Gewicht des Rückstandes der Probe. Die Abkürzung $m_{RK}$ steht für das Gewicht des Rückstandes der Kontrolle. Diese bestand nur aus einem Hydrogelstück und wurde in gleicher Weise behandelt wie die Probe. Die Abkürzung $m_{HAS}$ steht schließlich für das Gewicht der Hyaluronsäure-haltigen Schicht der Probe.

iv. Um zu kontrollieren, dass der Rückstand kein Wasser mehr enthielt und im Wesentlichen aus Hyaluronsäure bestand, wurde nach dem Wiegeschritt ein IR-Spektrum des Rückstandes angefertigt.

**Figur 11** zeigt das IR-Spektrum der verwendeten Hyaluronsäure (Referenzspektrum). **Figur 12** zeigt dann beispielhaft das IR-Spektrum eines Rückstandes. Die Ähnlichkeit der beiden Spektren wies darauf hin, dass der Rückstand im Wesentlichen aus Hyaluronsäure bestand.

**Figur 13** und **14** zeigen beispielhaft Rückstände der Proben. Wie in den Figuren zu sehen ist, lagen diese als transparente Filme vor, die auch vom Boden der Petrischalen abgezogen werden konnten (siehe insbesondere **Figur 14**). Das Vorhandensein sowie die Erscheinungsform dieser filmartigen Rückstände in Verbindung mit den Ergebnissen der IR-spektroskopischen Untersuchungen belegte, dass die Proben die Hyaluronsäure bei Kontakt mit einer wässrigen Flüssigkeit abgeben können.

In **Figur 15** sind die Versuchsergebnisse zur Hyaluronsäurefreisetzung als Mittelwerte von zwei bis drei verschiedenen Proben pro Inkubationszeit dargestellt. So haben die Versuche gezeigt, dass die hergestellten Proben nach einem Tag bereits fast 50 % und nach drei Tagen über 70 % der Hyaluronsäure freigesetzt haben. Eine höhere Hyaluronsäurefreisetzung konnte mit den gewählten Inkubationszeiten von bis zu 7 Tagen nicht beobachtet werden. Dies könnte die folgenden zwei Ursachen haben. Erstens das gegebenenfalls in den Hyaluronsäure-haltigen Schichten der Proben enthaltene Wasser, aufgrund dessen bei der vorliegenden Versuchsdurchführung die Werte für die Hyaluronsäurefreisetzung tendenziell zu niedrig berechnet werden können. Zweitens der gegebenenfalls unlösbar mit der Hydrogelschicht verbundene Anteil der Hyaluronsäure, welcher durch die Reaktion des Präpolymers mit der Hydrogelschicht und der Hyaluronsäure-haltigen Schicht ausgebildet werden kann (siehe vorangehenden Punkt 1iv). Jedenfalls haben die durchgeführten Versuche gezeigt, dass die Proben die enthaltene Hyaluronsäure in für die Wundheilung geeigneter Geschwindigkeit und Menge abgeben können. Die hergestellten Hyaluronsäure-haltigen Schichten können sich somit zumindest teilweise auflösen und als Opferschicht fungieren.

3. Absorptionskapazität der Proben

i. Eine wie zuvor beschrieben hergestellte Probe wurde in ein mit demineralisiertem Wasser gefülltes Becherglas gelegt und für 4 bis 72 Stunden bei Raumtemperatur inkubiert. Dabei befand sich die Probe stets unterhalb der Wasseroberfläche.
ii. Nach Ablauf der Inkubationszeit wurde die Probe aus dem Becherglas entfernt und gewogen. Die Absorptionskapazität der Probe in Prozent ($AK_P$) wurde dann mit folgender Formel berechnet:

$$AK_P = (m_{PNI} / m_{PVI}) * 100$$

Die Abkürzung $m_{PNI}$ bezeichnet dabei das Gewicht der Probe nach der Inkubation. Die Abkürzung $m_{PVI}$ bezeichnet hingegen das Gewicht der Probe vor der Inkubation.

**Figur 16** zeigt die ermittelten Absorptionskapazitäten, bei denen es sich ebenso wie zuvor bei den Freisetzungsuntersuchungen um Mittelwerte von zwei bis drei verschiedenen Proben pro Inkubationszeit handelt. Die Proben konnten demnach mehr als das Dreifache ihres Eigengewichtes an Wasser absorbieren. Der Rückgang in der Absorption vom 48 Stundenwert auf den 72 Stundenwert hat möglicherweise damit zu tun, dass sich die Hyaluronsäure-haltige Schicht der Probe mit zunehmender Inkubationszeit immer mehr auflöst und dieser Prozess einer anderen Kinetik als die Wasseraufnahme durch das Hydrogel folgt. So ist denkbar, dass die Probe beziehungsweise die Hydrogelschicht der Probe nach 48 Stunden ihre maximale Absorptionskapazität erreicht hat, die Hyaluronsäure-haltige Schicht der Probe zu diesem Zeitpunkt aber noch nicht vollständig aufgelöst war (**Figur 15** bestätigt dies auch). Somit würde die Probe - wie beobachtet - bei längerer Inkubation wieder an Gewicht verlieren. Dies bedeutet jedoch nicht, dass die Hydrogelschicht Wasser nur für eine begrenzte Zeit binden kann, sondern geht vielmehr auf den Gewichtsverlust infolge der sich auflösenden Hyaluronsäure-haltigen Opferschicht zurück.

4. Einfluss der Proben auf den pH-Wert

i. Eine wie zuvor beschrieben hergestellte Probe wurde in ein mit demineralisiertem Wasser gefülltes Becherglas gelegt und für 4 bis 72 Stunden bei Raumtemperatur inkubiert. Dabei befand sich die Probe stets unterhalb der Wasseroberfläche. Das für den Test verwendete Wasser hatte einen pH-Wert von 6,1.
ii. Es wurde dann der pH-Wert der im Becherglas enthaltenen Flüssigkeit bestimmt.

Die Ergebnisse der pH-Messungen sind in **Figur 17** dargestellt. Auch hier handelt es sich um Mittelwerte von zwei bis drei verschiedenen Proben je Inkubationszeitpunkt. Die gemessenen pH-Werte liegen allesamt weiterhin im leicht sauren Bereich, was vorteilhaft für die Wundheilung sein kann.

## Patentansprüche

**1.** Wundauflage (1, 5, 7, 11, 13) umfassend

- eine Hydrogelschicht (2) und
- eine Wundkontaktschicht (3), welche mit einer wundzugewandten Seite der Hydrogelschicht (2) verbunden ist und Hyaluronsäure umfasst,

**dadurch gekennzeichnet, dass**
die Wundkontaktschicht (3) filmartig ausgebildet ist und das Material, aus dem die Wundkontaktschicht (3) besteht, nicht porös ausgebildet ist.

**2.** Wundauflage nach Anspruch 1, **dadurch gekennzeichnet, dass** die Hydrogelschicht (2) ein Polyurethan-Polymer,

insbesondere ein Polyurethan-Polyharnstoff-Copolymer, umfasst.

3. Wundauflage nach Anspruch 1 oder 2, **dadurch gekennzeichnet, dass** die Hydrogelschicht (2) erhältlich ist durch Umsetzung eines Amin-terminierten Präpolymers enthaltend Polyalkylenoxideinheiten mit einem Isocyanat-terminierten Präpolymer enthaltend Polyalkylenoxideinheiten in Gegenwart von Wasser und optional eines mehrwertigen Alkohols, insbesondere Glycerol.

4. Wundauflage nach einem der vorangehenden Ansprüche, **dadurch gekennzeichnet, dass** die Hydrogelschicht (2) perforiert ist.

5. Wundauflage nach einem der vorangehenden Ansprüche, **dadurch gekennzeichnet, dass** die Hyaluronsäure ein molekulares Gewicht von 1000 bis 1800 kDa oder von mehr als 1800 kDa aufweist.

6. Wundauflage nach einem der vorangehenden Ansprüche, **dadurch gekennzeichnet, dass** die Wundkontaktschicht (3) als Opferschicht ausgebildet ist.

7. Wundauflage nach einem der vorangehenden Ansprüche, **dadurch gekennzeichnet, dass** die Wundkontaktschicht eine Hyaluronsäurefreisetzung von mindestens 50 %, bevorzugt von mindestens 60 %, mehr bevorzugt von mindestens 70 %, besonders bevorzugt von mindestens 80 % und ganz besonders bevorzugt von mindestens 90 % aufweist.

8. Wundauflage nach einem der vorangehenden Ansprüche, **dadurch gekennzeichnet, dass** die Wundkontaktschicht (3) erhältlich ist durch Trocknen einer flüssigen oder gelartigen Zubereitung umfassend Hyaluronsäure und Wasser.

9. Wundauflage nach Anspruch 8, **dadurch gekennzeichnet, dass** das Trocknen eine Gefriertrocknung und/oder eine Lufttrocknung umfasst.

10. Wundauflage nach Anspruch 8 oder 9, **dadurch gekennzeichnet, dass** die Zubereitung nur teilweise getrocknet ist, so dass ein Rest Wasser in der Wundkontaktschicht (3) vorhanden ist.

11. Wundauflage nach einem der Ansprüche 8 bis 10, **dadurch gekennzeichnet, dass** die Zubereitung aus Hyaluronsäure und Wasser besteht.

12. Wundauflage nach einem der vorangehenden Ansprüche, **dadurch gekennzeichnet, dass** die Wundkontaktschicht (3) perforiert ist, wobei - falls die Hydrogelschicht (2) gleichfalls perforiert ist - sich die Perforationen in der Hydrogelschicht (2) und der Wundkontaktschicht (3) vorzugsweise überlagern.

13. Wundauflage nach einem der vorangehenden Ansprüche, **dadurch gekennzeichnet, dass** die Wundkontaktschicht (3) mit der Hydrogelschicht (2) verbunden ist, indem die Schichten miteinander in Kontakt gebracht werden, wenn die Hydrogelschicht (2) noch nicht vollständig polymerisiert ist.

14. Wundauflage nach einem der Ansprüche 1 bis 12, **dadurch gekennzeichnet, dass** die Wundkontaktschicht (3) mit der Hydrogelschicht (2) durch ein Verbindungsmittel verbunden ist, wobei das Verbindungsmittel vorzugsweise ein Isocyanat-terminiertes Präpolymer enthaltend Polyalkylenoxideinheiten ist.

15. Wundauflage nach Anspruch 14, **dadurch gekennzeichnet, dass** zur Herstellung der Hydrogelschicht (2) ein Isocyanat-terminiertes Präpolymer enthaltend Polyalkylenoxideinheiten verwendet wird, wobei zur Herstellung der Hydrogelschicht (2) und zur Verbindung der Wundkontaktschicht (3) mit der Hydrogelschicht (2) das gleiche Isocyanat-terminierte Präpolymer verwendet wird.

16. Wundauflage nach einem der vorangehenden Ansprüche, **dadurch gekennzeichnet, dass** die Wundauflage weiterhin eine Rückschicht (4, 9) umfasst, wobei die Rückschicht (4, 9) vorzugsweise eine wasserundurchlässige und wasserdampfdurchlässige Kunststofffolie, insbesondere Polyurethanfolie, umfasst.

17. Wundauflage nach einem der vorangehenden Ansprüche, **dadurch gekennzeichnet, dass** die Wundauflage weiterhin eine absorbierende Schicht (12), insbesondere eine absorbierende Schaumstoffschicht, umfasst.

18. Wundauflage nach einem der vorangehenden Ansprüche zur Anwendung in der Behandlung von Wunden am

menschlichen oder tierischen Körper in der Granulations- oder Epithelisierungsphase.

**19.** Verfahren zur Herstellung einer Wundauflage nach einem der Ansprüche 1 bis 17 umfassend die Schritte

i. Bereitstellen einer Hydrogelschicht (2),
ii. Bereitstellen einer Wundkontaktschicht (3) umfassend Hyaluronsäure,
iii. Verbinden der Wundkontaktschicht (3) mit einer wundzugewandten Seite der Hydrogelschicht (2).

## Claims

**1.** Wound dressing (1, 5, 7, 11, 13) comprising

- a hydrogel layer (2) and
- a wound contact layer (3) which is connected to a wound-facing side of the hydrogel layer (2) and comprises hyaluronic acid, **characterized in that**

the wound contact layer (3) is film-like and the material of which the wound contact layer (3) consists is not porous.

**2.** Wound dressing according to Claim 1, **characterized in that** the hydrogel layer (2) comprises a polyurethane polymer, more particularly a polyurethane-polyurea copolymer.

**3.** Wound dressing according to Claim 1 or 2, **characterized in that** the hydrogel layer (2) is obtainable by reacting an amine-terminated prepolymer containing polyalkylene oxide units with an isocyanate-terminated prepolymer containing polyalkylene oxide units in the presence of water and optionally a polyhydric alcohol, more particularly glycerol.

**4.** Wound dressing according to any of the preceding claims, **characterized in that** the hydrogel layer (2) is perforated.

**5.** Wound dressing according to any of the preceding claims, **characterized in that** the hyaluronic acid has a molecular weight of 1000 to 1800 kDa or of more than 1800 kDa.

**6.** Wound dressing according to any of the preceding claims, **characterized in that** the wound contact layer (3) is configured as a sacrificial layer.

**7.** Wound dressing according to any of the preceding claims, **characterized in that** the wound contact layer exhibits a hyaluronic acid release of at least 50%, preferably of at least 60%, more preferably of at least 70%, very preferably of at least 80% and especially preferably of at least 90%.

**8.** Wound dressing according to any of the preceding claims, **characterized in that** the wound contact layer (3) is obtainable by drying a liquid or gel-like preparation comprising hyaluronic acid and water.

**9.** Wound dressing according to Claim 8, **characterized in that** the drying comprises a freeze drying and/or an air drying.

**10.** Wound dressing according to Claim 8 or 9, **characterized in that** the preparation is only partially dried, and so a residue of water is present in the wound contact layer (3).

**11.** Wound dressing according to any of Claims 8 to 10, **characterized in that** the preparation consists of hyaluronic acid and water.

**12.** Wound dressing according to any of the preceding claims, **characterized in that** the wound contact layer (3) is perforated, wherein - if the hydrogel layer (2) is likewise perforated - the perforations in the hydrogel layer (2) and in the wound contact layer (3) preferably overlap one another.

**13.** Wound dressing according to any of the preceding claims, **characterized in that** the wound contact layer (3) is connected to the hydrogel layer (2) by bringing the layers into contact with one another when the hydrogel layer (2) is not yet completely polymerized.

**14.** Wound dressing according to any of Claims 1 to 12, **characterized in that** the wound contact layer (3) is connected to

the hydrogel layer (2) by a connecting agent, wherein the connecting agent is preferably an isocyanate-terminated prepolymer containing polyalkylene oxide units.

**15.** Wound dressing according to Claim 14, **characterized in that** for producing the hydrogel layer (2) an isocyanate-terminated prepolymer containing polyalkylene oxide units is used, wherein the same isocyanate-terminated prepolymer is used for producing the hydrogel layer (2) and for connecting the wound contact layer (3) to the hydrogel layer (2).

**16.** Wound dressing according to any of the preceding claims, **characterized in that** the wound dressing further comprises a backing layer (4, 9), wherein the backing layer (4, 9) preferably comprises a water-impermeable and water vapour-permeable plastics foil, more particularly polyurethane foil.

**17.** Wound dressing according to any of the preceding claims, **characterized in that** the wound dressing further comprises an absorbent layer (12), more particularly an absorbent foam layer.

**18.** Wound dressing according to any of the preceding claims for use in the treatment of wounds on the human or animal body in the granulation or epithelialization phase.

**19.** Method for producing a wound dressing according to any of Claims 1 to 17, comprising the steps of

i. providing a hydrogel layer (2),
ii. providing a wound contact layer (3) comprising hyaluronic acid,
iii. connecting the wound contact layer (3) to a wound-facing side of the hydrogel layer (2).

**Revendications**

**1.** Pansement (1, 5, 7, 11, 13) comprenant

- une couche d'hydrogel (2) et
- une couche (3) en contact avec la plaie, qui est liée à une face tournée vers la plaie de la couche d'hydrogel (2) et comprend de l'acide hyaluronique,

**caractérisé en ce que**
la couche (3) en contact avec la plaie est conçue sous forme de film et la matière à partir de laquelle est constituée la couche (3) en contact avec la plaie est conçue non poreuse.

**2.** Pansement selon la revendication 1, **caractérisé en ce que** la couche d'hydrogel (2) comprend un polymère polyuréthane, en particulier un copolymère polyuréthane-polyurée.

**3.** Pansement selon la revendication 1 ou 2, **caractérisé en ce que** la couche d'hydrogel (2) peut être obtenue par mise en réaction d'un prépolymère à terminaison amine, contenant des unités polyoxyalkylène, avec un prépolymère à terminaison isocyanate, contenant des unités polyoxyalkylène, en présence d'eau et en option d'un alcool polyhydrique, en particulier de glycérol.

**4.** Pansement selon l'une quelconque des revendications précédentes, **caractérisé en ce que** la couche d'hydrogel (2) est perforée.

**5.** Pansement selon l'une quelconque des revendications précédentes, **caractérisé en ce que** l'acide hyaluronique présente une masse moléculaire de 1 000 à 1 800 kDa ou de plus de 1 800 kDa.

**6.** Pansement selon l'une quelconque des revendications précédentes, **caractérisé en ce que** la couche (3) en contact avec la plaie est conçue sous forme de couche sacrificielle.

**7.** Pansement selon l'une quelconque des revendications précédentes, **caractérisé en ce que** la couche en contact avec la plaie présente une libération d'acide hyaluronique d'au moins 50 %, de préférence d'au moins 60 %, encore mieux d'au moins 70 %, de façon particulièrement préférée d'au moins 80 % et de façon tout particulièrement préférée d'au moins 90 %.

8. Pansement selon l'une quelconque des revendications précédentes, **caractérisé en ce que** la couche (3) en contact avec la plaie peut être obtenue par séchage d'une préparation liquide ou de type gel, comprenant de l'acide hyaluronique et de l'eau.

9. Pansement selon la revendication 8, **caractérisé en ce que** le séchage comprend une lyophilisation et/ou un séchage à l'air.

10. Pansement selon la revendication 8 ou 9, **caractérisé en ce que** la préparation n'est que partiellement séchée, de sorte qu'un reste d'eau est présent dans le couche (3) en contact avec la plaie.

11. Pansement selon l'une quelconque des revendications 8 à 10, **caractérisé en ce que** la préparation est constituée d'acide hyaluronique et d'eau.

12. Pansement selon l'une quelconque des revendications précédentes, **caractérisé en ce que** la couche (3) en contact avec la plaie est perforée, les perforations dans la couche d'hydrogel (2) - dans le cas où la couche d'hydrogel (2) est également perforée - et dans la couche (3) en contact avec la plaie se superposant.

13. Pansement selon l'une quelconque des revendications précédentes, **caractérisé en ce que** la couche (3) en contact avec la plaie est liée à la couche d'hydrogel (2), par le fait que les couches sont mises en contact entre elles alors que la couche d'hydrogel (2) n'est pas encore totalement polymérisée.

14. Pansement selon l'une quelconque des revendications 1 à 12, **caractérisé en ce que** la couche (3) en contact avec la plaie est liée à la couche d'hydrogel (2) par un agent de liaison, l'agent de liaison étant de préférence un polymère à terminaison isocyanate, contenant des unités polyoxyalkylène.

15. Pansement selon la revendication 14, **caractérisé en ce que** pour la préparation de la couche d'hydrogel (2) est utilisé un prépolymère à terminaison isocyanate, contenant des unités polyoxyalkylène, le même prépolymère à terminaison isocyanate étant utilisé pour la préparation de la couche d'hydrogel (2) et pour la liaison de la couche (3) en contact avec la plaie avec la couche d'hydrogel (2).

16. Pansement selon l'une quelconque des revendications précédentes, **caractérisé en ce que** le pansement comprend en outre une couche de dos (4, 9), la couche de dos (4, 9) comprenant de préférence un film en matière plastique imperméable à l'eau et imperméable à la vapeur d'eau, en particulier un film de polyuréthane.

17. Pansement selon l'une quelconque des revendications précédentes, **caractérisé en ce que** le pansement comprend en outre une couche absorbante (12), en particulier une couche de mousse absorbante.

18. Pansement selon l'une quelconque des revendications précédentes, pour utilisation dans le traitement de plaies sur le corps humain ou animal, dans la phase de granulation ou d'épithélisation.

19. Procédé pour la fabrication d'un pansement selon l'une quelconque des revendications 1 à 17, comprenant les étapes

   i. disposition d'une couche d'hydrogel (2),
   ii. disposition d'une couche (3) en contact avec la plaie, comprenant de l'acide hyaluronique,
   iii. liaison de la couche (3) en contact avec la plaie, avec une face tournée vers la plaie de la couche d'hydogel (2) .

Figur 1

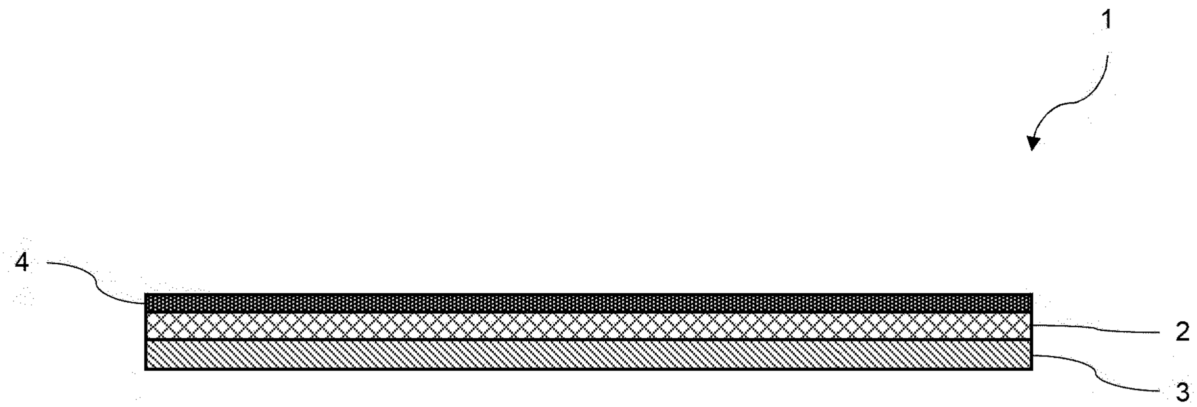


Figur 2

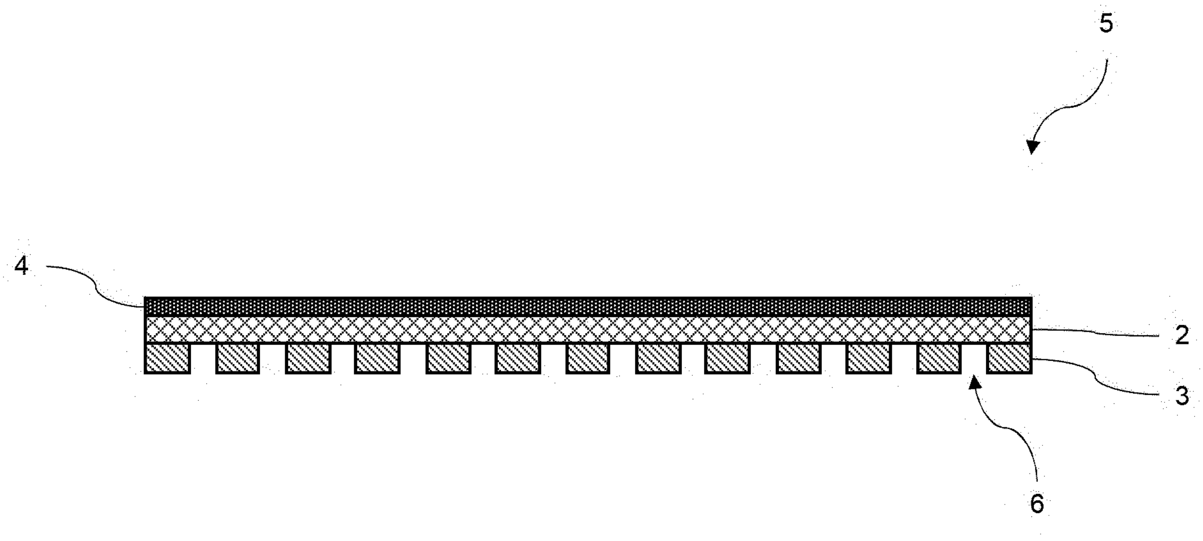

Figur 3

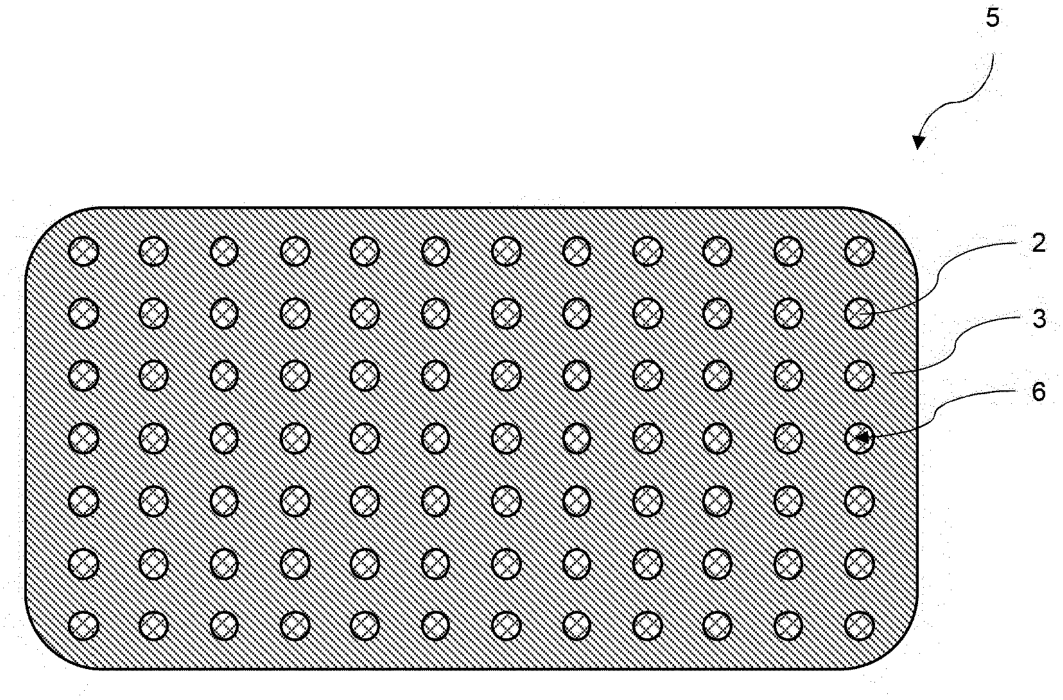


Figur 4

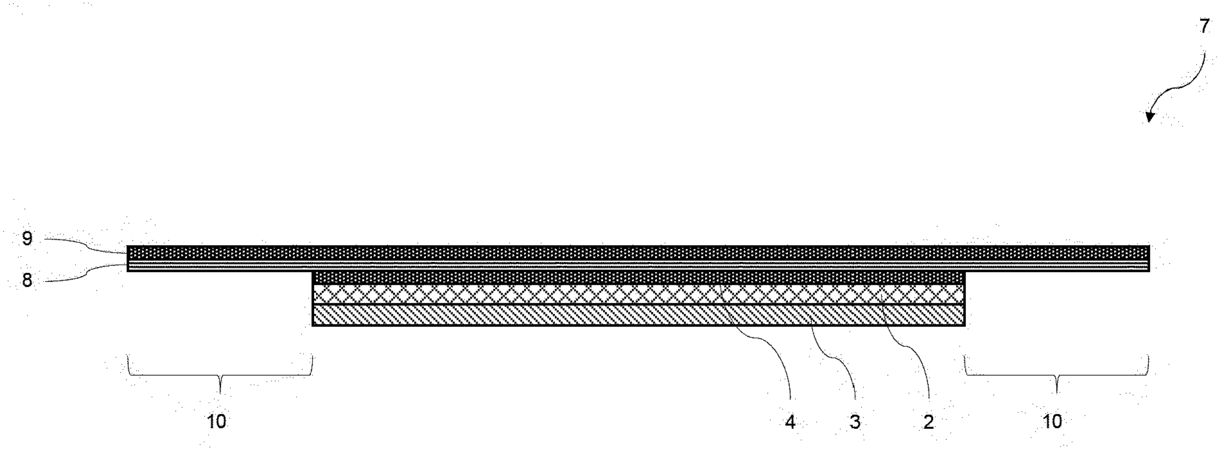

Figur 5

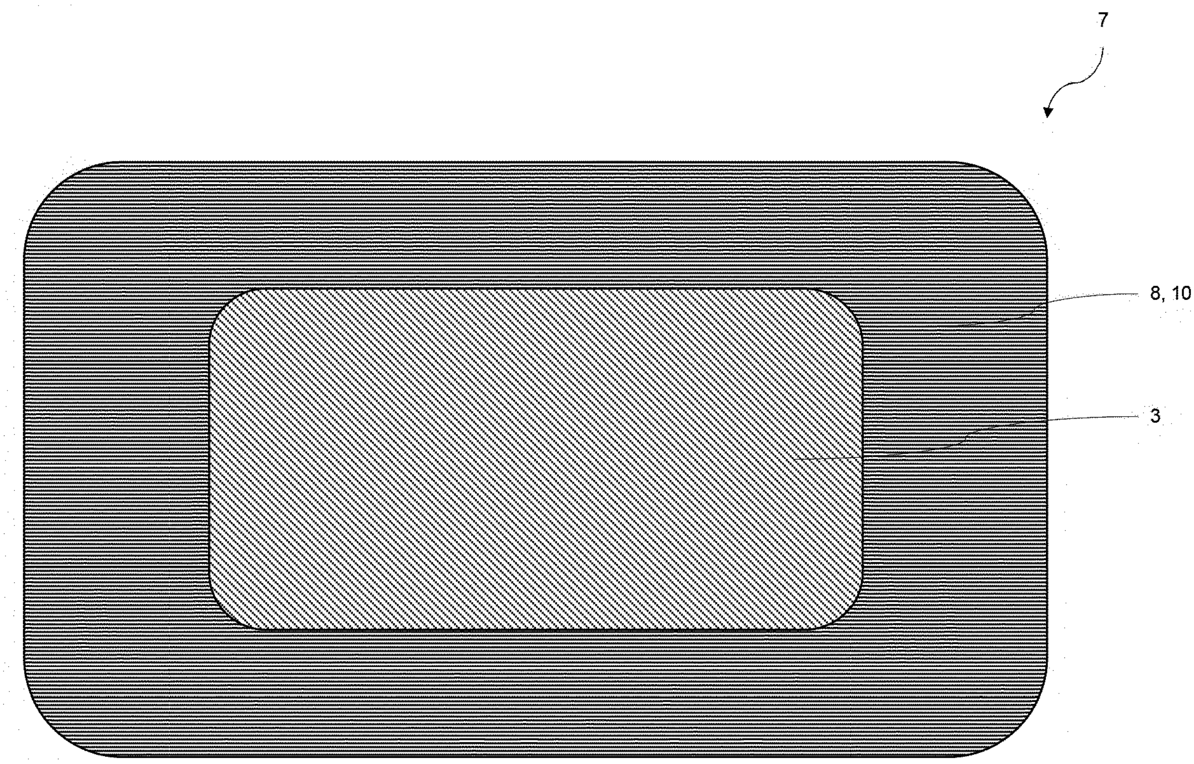


Figur 6

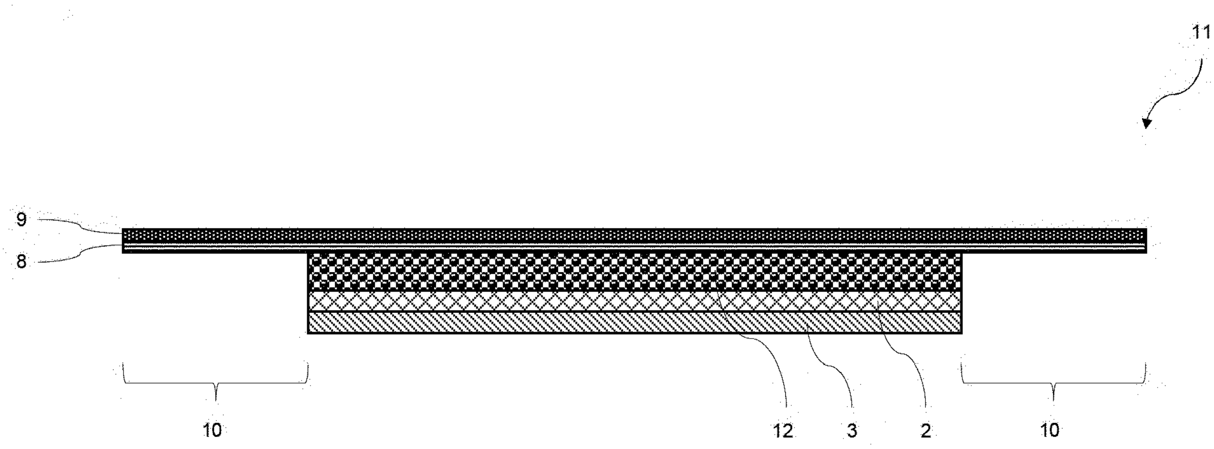

Figur 7

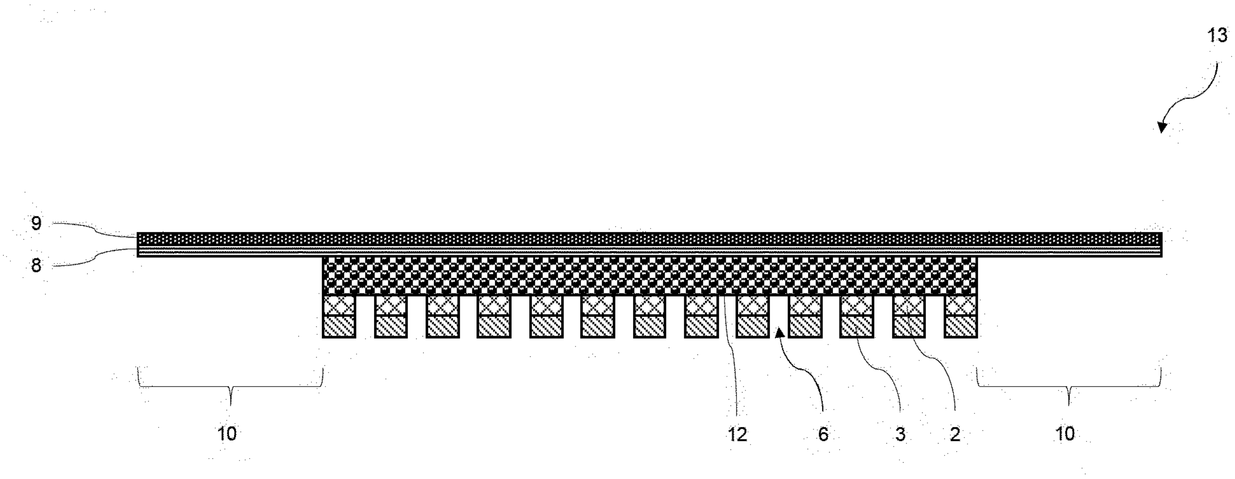


Figur 8

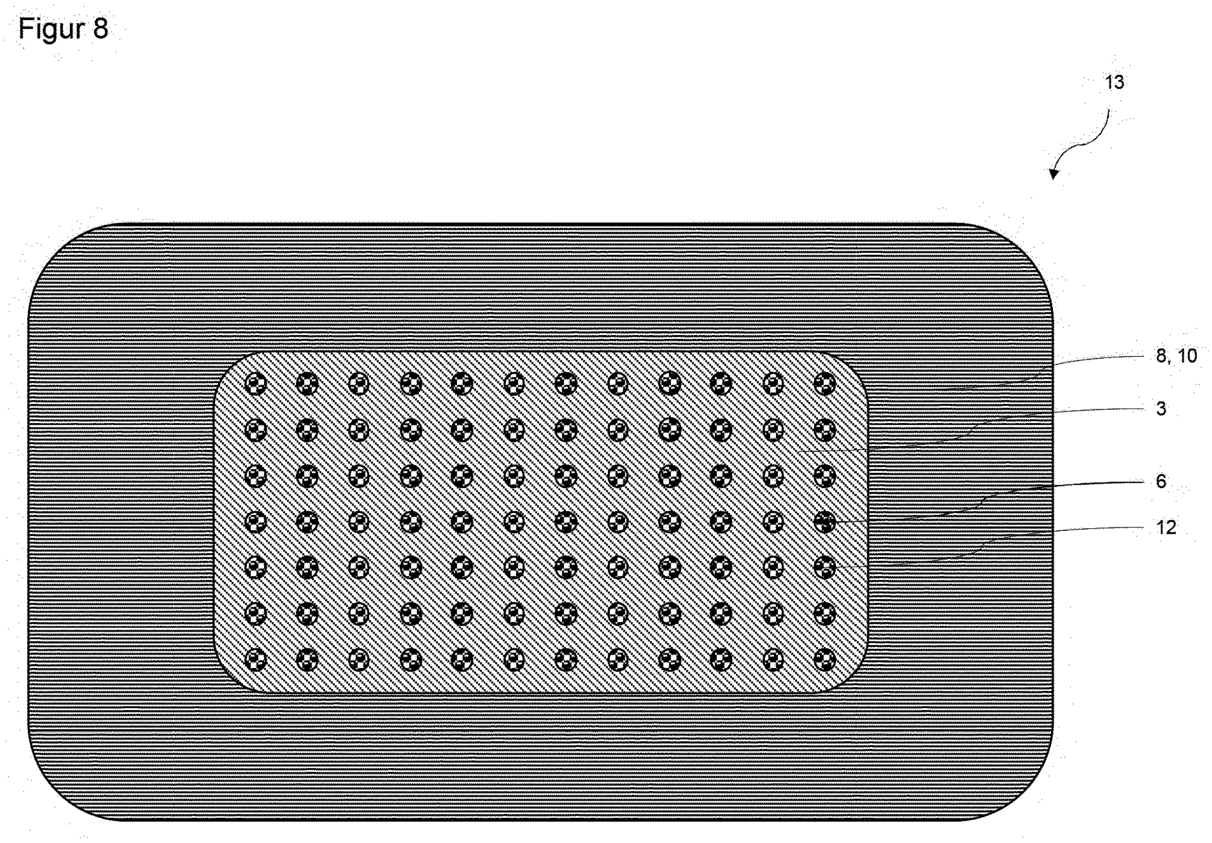

Figur 9

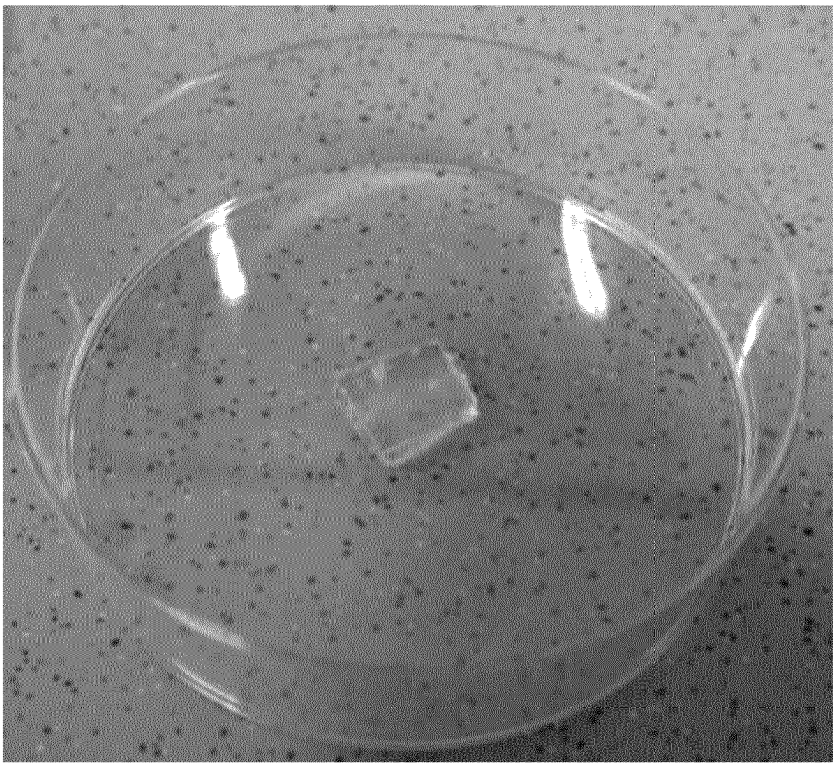

Figur 10

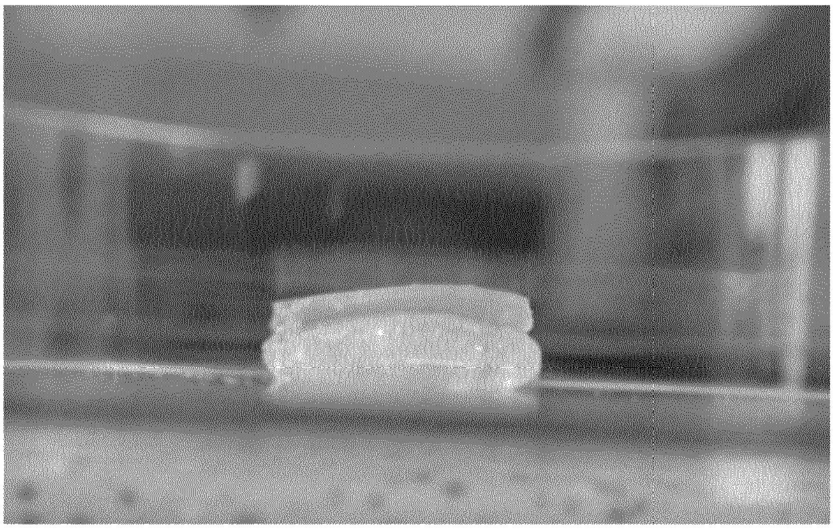

Figur 11

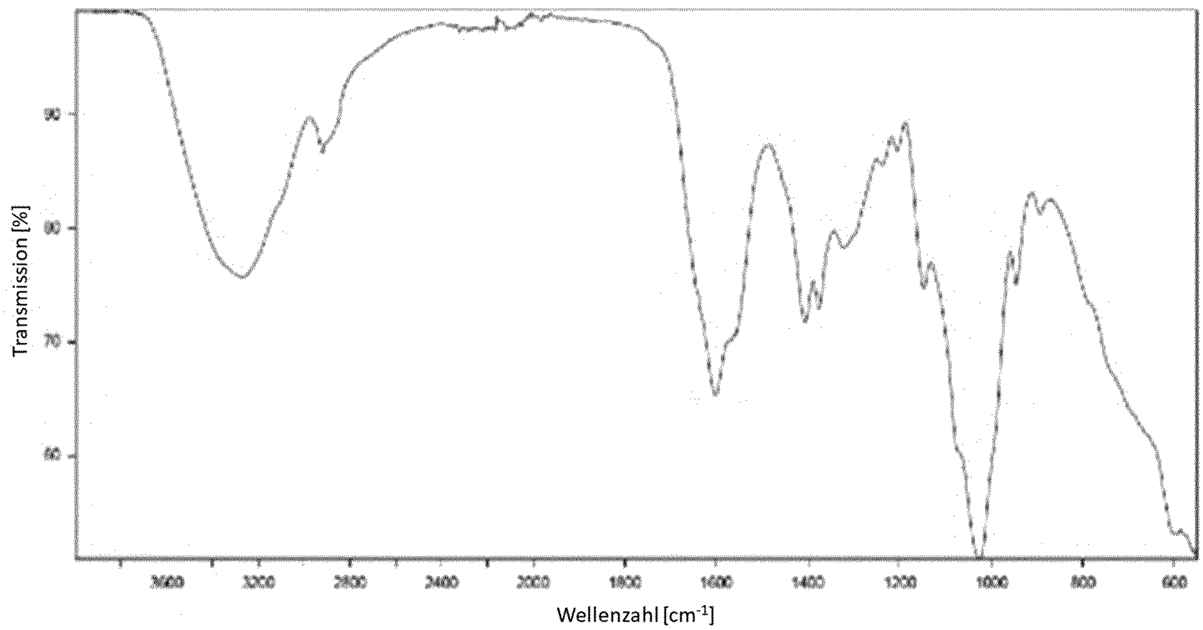


Figur 12

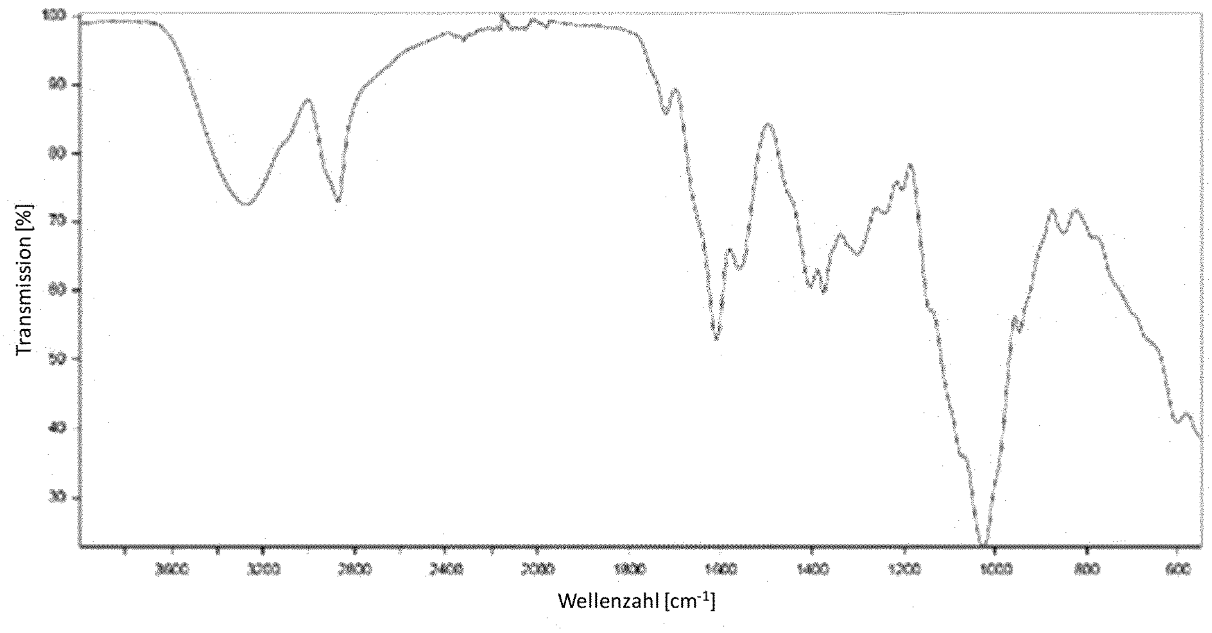

Figur 13

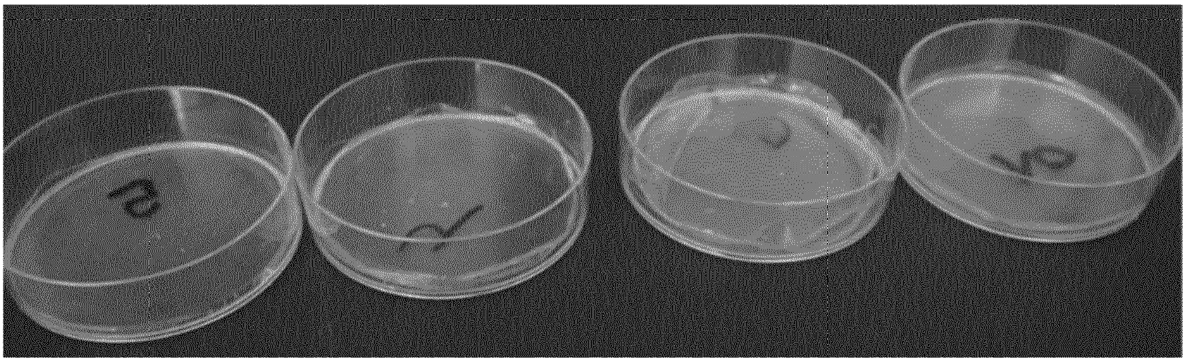

Figur 14

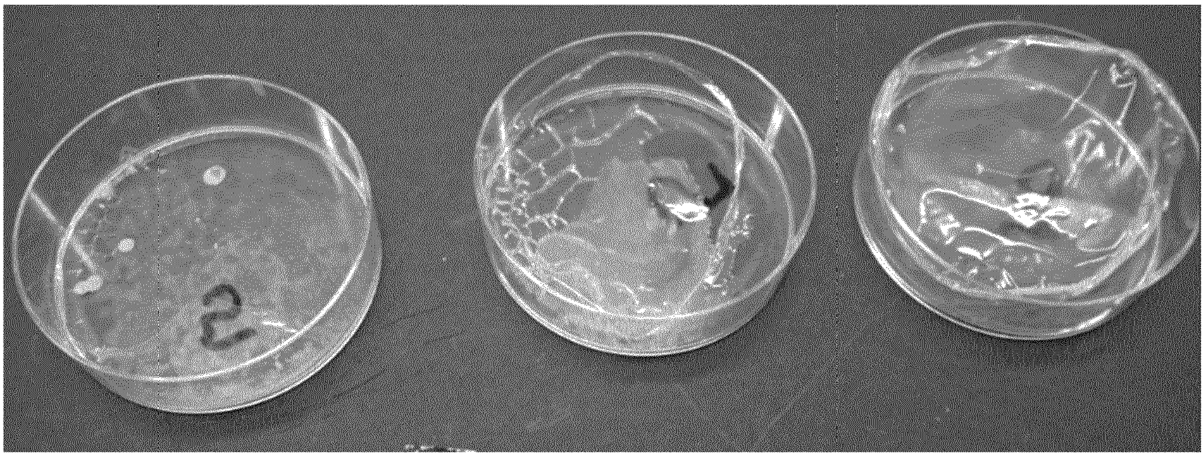

Figur 15

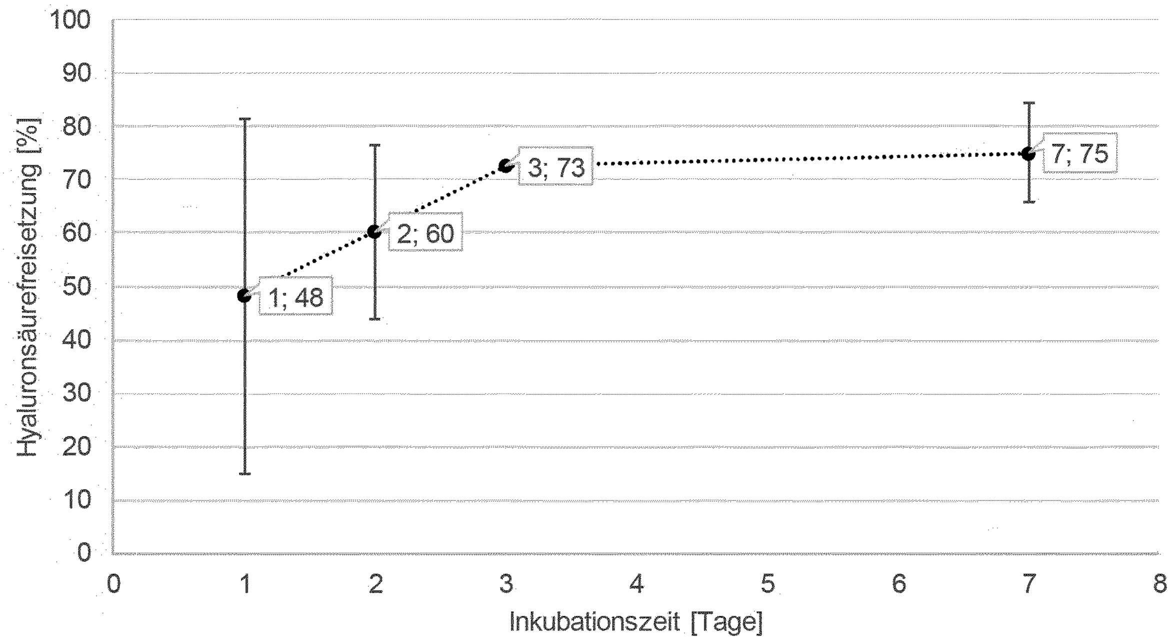


Figur 16

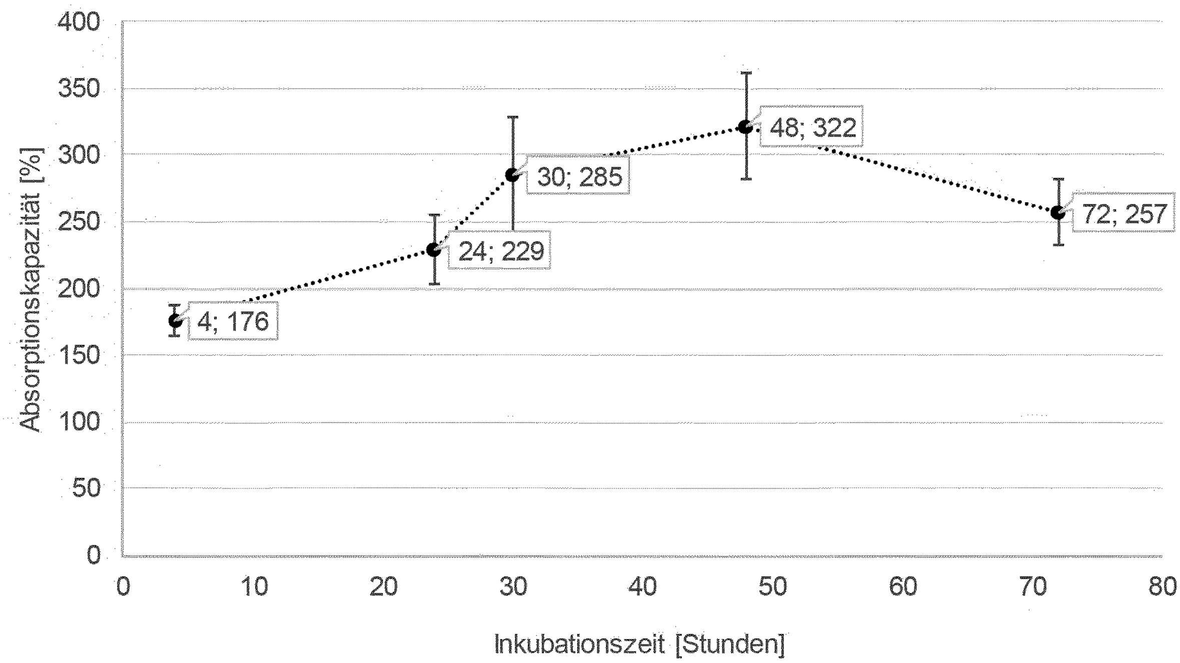

Figur 17

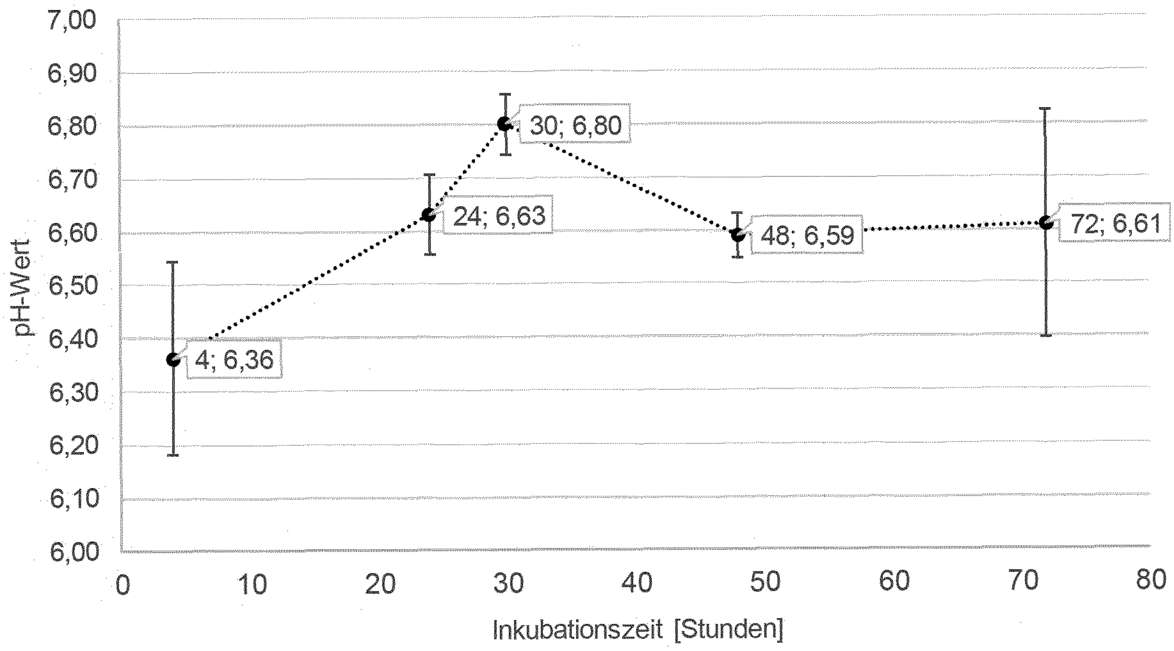
7,00
6,90
6,80
6,70
6,60
6,50
6,40
6,30
6,20
6,10
6,00
pH-Wert
0
10
20
30
40
50
60
70
80
Inkubationszeit [Stunden]
4; 6,36
24; 6,63
30; 6,80
48; 6,59
72; 6,61

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2010000451 A1
- WO 2018115257 A1
- EP 3587456 A1
- US 20020111576 A1

### In der Beschreibung aufgeführte Nicht-Patentliteratur

- Hyaluronan in wound healing: Rediscovering a major player. *Wound Rep Reg*, 2014, vol. 22, 579-593